## Europäisches Patentamt
## European Patent Office
## Office européen des brevets

(19)

(11) Veröffentlichungsnummer: **0 106 146 B1**

(12)

# EUROPÄISCHE PATENTSCHRIFT

(45) Veröffentlichungstag der Patentschrift:
**12.03.86**

(21) Anmeldenummer: **83108960.2**

(22) Anmeldetag: **10.09.83**

(51) Int. Cl.⁴: **C 12 P 21/02**, C 12 N 9/26, C 12 N 9/80

(54) Verfahren zur biologischen Herstellung von Glutathion.

(30) Priorität: **13.10.82 DE 3237896**

(43) Veröffentlichungstag der Anmeldung:
**25.04.84 Patentblatt 84/17**

(45) Bekanntmachung des Hinweises auf die Patenterteilung:
**12.03.86 Patentblatt 86/11**

(84) Benannte Vertragsstaaten:
**DE FR GB IT NL**

(56) Entgegenhaltungen:
**EP - A - 0 042 306
EP - A - 0 058 426
DE - B - 2 845 387
US - A - 4 032 407
US - A - 4 046 921**

**CHEMICAL ABSTRACTS, Band 95, Nr. 17, 26. Oktober 1981, Seite 497, Nr. 148671b, Columbus, Ohio, USA O. MOEBUS et al.: "Growth of Saccharomyces cerevisiae in form of solid particles in a gaseous fluidized bed"
CHEMICAL ABSTRACTS, Band 80, Nr. 23, 10. Juni 1974, Seite 345, Nr. 131676z, Columbus, Ohio, USA
CHEMICAL ABSTRACTS, Band 77, Nr. 5, 31. Juli 1972, Seite 379, Nr. 32747e, Columbus, Ohio, USA
CHEMICAL ABSTRACTS, Band 74, Nr. 13, 29. März 1971, Seite 220, Nr. 63186p, Columbus, Ohio, USA**

(73) Patentinhaber: **HÜLS AKTIENGESELLSCHAFT, Patentabteilung / PB 15 - Postfach 13 20, D-4370 Marl 1 (DE)**

(72) Erfinder: **Thommel, Jürgen, Dr., Lohe 4 a, D-2071 Delingsdorf (DE)**
Erfinder: **Kirk, Hans-Georg, Dr., Spannwisch 7, D-2000 Hamburg 72 (DE)**
Erfinder: **Hill, Frank F., Dr., Schlesienstrasse 23, D-4020 Mettmann-Obschwarzbach (DE)**

(56) Entgegenhaltungen: (Fortsetzung)
**CHEMICAL ABSTRACTS, Band 65, Nr. 11, 21. November 1966, Spalte 17663C, Columbus, Ohio, USA
THE YEASTS, Band 3, 1970, Seiten 529-545, Academic Press, London, G.B. J.S. HARRISON: "Miscellaneous products from yeast"**

## Beschreibung

Die Erfindung betrifft ein Verfahren zur biologischen Herstellung von Glutathion durch fermentative Umsetzung von Präkursor-Verbindungen mittels Hefe.

Die Erfindung bezweckt, die Ausbeute an Glutathion zu erhöhen und diese Substanz in einer Form zu erzeugen, in der sie direkt weiterverwendet werden kann oder aus der sie in vergleichsweise einfachen Verfahren abgetrennt und gereinigt werden kann.

Die Umsetzung von Präkursor-Verbindungen zu halbsynthetischen Produkten mittels Mikroorganismen ist bekannt. Dabei werden unter Präkursoren solche Verbindungen verstanden, die von geeigneten Mikroorganismen in wenigen enzymatischen Umwandlungsschritten zu Folgeprodukten umgesetzt werden. Die Folgeprodukte sind vorzugsweise pharmazeutisch einsetzbare wertvolle Substanzen, die durch chemisch-synthetische Methoden nur schwer oder bisher gar nicht zugänglich sind.

Nach DE-PS Nr. 548459 kann beispielsweise Benzaldehyd zu Phenylacetylcarbinol fermentativ umgesetzt werden, woraus man mittels weiterer chemischer Umwandlungen das pharmazeutisch wertvolle Ephedrin erhält. Nach US Nr. 3281407 kann man durch fermentative Verknüpfung von Cystein, Glutaminsäure und Glycin in verdünnter wässeriger Phase das pharmazeutisch wichtige Peptid Glutathion herstellen und durch Extrahieren von den abgetöteten Hefezellen trennen. Nach JP Nr. 50031546 kann man mittels Hefe aus 5'-Cytidylsäure und Cholin das Pharmazeutikum Cytidindiphosphorcholin herstellen.

In EP-A-O Nr. 0058426 wird ein Verfahren zur schlempefreien Herstellung von Äthanol und Einzellerprotein in einem mit Gas fluidisierten Wirbelbett beschrieben. Auf die fluidisierten Partikel (z.B. Sacch. cerev.) wird eine Nährlösung mit fermentierbaren Kohlenhydraten aufgesprüht. Das mit Wasser abgedampfte Äthanol wird in einem nachgeschalteten Kondensator abgeschieden. Das Wirbelbett dient sowohl zur Vermehrung der Mikroorganismen als auch gleichzeitig zur Bildung von Äthanol.

Versetzt man die Biomasse mit geeigneten Effektor-Verbindungen, so kann man die Reaktionsgeschwindigkeit einer Enzymreaktion erhöhen und dadurch grössere Konzentrationen der gewünschten Verbindungen in der Biomasse erreichen.

Die obengenannten und andere entsprechende Umsetzungen werden üblicherweise in verdünnter wässeriger Phase als submerse Fermentation durchgeführt. In der Stoffwechselphysiologie der Mikroorganismen ist die Erzeugungsrate von Metaboliten und Enzymen im allgemeinen stark von der Konzentration der anwesenden Präkursoren oder Effektoren abhängig, und zwar nimmt in den meisten Fällen die Erzeugungsrate mit der Konzentration zu.

Bei der submersen Fermentation besteht der Reaktionsansatz meist zu mehr als 90% aus Wasser.

Deshalb müssen hierbei die Präkursoren oder Effektoren in relativ grosser Menge eingesetzt werden, um eine hinreichend grosse Konzentration der Präkursoren oder Effektoren — bezogen auf die Biomasse — und dadurch eine annehmbare Ausbeute an gewünschten Metaboliten oder Enzymen zu erhalten.

Damit stellt sich die Aufgabe, ein Verfahren zur biologischen Herstellung von Glutathion mittels Hefe unter Verwendung von L-Cystein, L-Glutaminsäure und Glycin als Präkursoren zu finden, bei dem die Hefe in grösserer Konzentration vorliegt als bei der submersen Fermentation, wobei jedoch die Bedingungen, unter denen sich die Hefe befindet, auf deren Anforderungen abgestimmt werden können, damit die Stoffwechselvorgänge in der gewünschten Richtung ablaufen.

Diese Aufgabe wird erfindungsgemäss gelöst durch ein Verfahren, bei dem die Hefe (Sacch. cerev.) nach einer bekannten Methode granuliert wird, in Form wirbelfähiger Partikel in einem Wirbelbettreaktor mittels Luft fluidisiert wird, mit einer wässerigen Lösung der Präkursoren besprüht wird und weiter mittels Luft bei einer Temperatur von 20 bis 45° C und einer Verweilzeit von 30 Minuten bis 5 Stunden im Wirbelbett fluidisiert wird zwecks Umsetzen der Präkursoren zu Glutathion.

Nach beendeter Umsetzung kann die Hefe entweder in Form der granulierten Partikel ohne weitere Aufarbeitung unmittelbar verwendet werden, oder sie kann durch Zusatz von Konservierungsmitteln im feuchten Zustand lagerfähig gemacht werden, oder sie kann durch anschliessendes Trocknen im Wirbelbett lagerfähig gemacht werden, oder das Glutathion kann nach bekannten Verfahren extrahiert werden, um es in reiner Form zu gewinnen.

Bei dem erfindungsgemässen Verfahren ist die Menge des Wassers, das die Hefe umgibt, wesentlich kleiner als bei der submersen Fermentation. Setzt man beispielsweise granulierte Hefe mit 35% Trockenmasse ein, ist ausserhalb der Hefezellen kaum noch Wasser vorhanden. Dadurch lässt sich eine relativ grosse Konzentration der Präkursoren auf der Hefe erreichen, wodurch diese fermentative Umsetzung beschleunigt wird.

Die zur Herstellung der wirbelfähigen Partikel verwendete Hefe wird nach bekannten Submers-Verfahren gezüchtet, aus der Kulturbrühe abgetrennt und anschliessend nach einem ebenfalls bekannten Verfahren, wie es beispielsweise in DE-OS Nr. 2813077 angegeben ist, zu wirbelfähigen Partikeln umgearbeitet. Hierbei können Zusatzstoffe oder Trägerstoffe zugegeben werden, die die Granulierung erleichtern oder ermöglichen.

Die wirbelfähigen Partikel werden im Wirbelbettreaktor durch einen Gasstrom — bevorzugt einen Luftstrom — und gegebenenfalls mittels mechanischer Bewegung fluidisiert. An Stelle von Luft kann auch Sauerstoff oder ein anderes sauerstoffhaltiges Gas verwendet werden.

Damit der Stoffwechsel in der lebenden Hefe nicht behindert wird, darf der zur Fluidisierung benutzte Gasstrom die Hefe nicht austrocknen. Deshalb wird der Gasstrom zweckmässigerweise mit Wasserdampf gesättigt, und man sprüht gegebenenfalls zusätzlich etwas Wasser auf die im Wirbelbett befindlichen Partikel. Mit diesem Sprühwasser kann man gleichzeitig den Präkursor auf die Partikel bringen.

Die bei dieser erfindungsgemässen fermentativen Umsetzung freiwerdende Wärmemenge ist gering, weil keine Wachstumsvorgänge ablaufen und praktisch keine neue Hefe erzeugt wird. Die gewünschte Temperatur im Wirbelbettreaktor kann in jedem Falle durch Wahl einer geeigneten Temperatur des zur Fluidisierung benutzten Gases eingehalten werden.

Das erfindungsgemässe Verfahren hat folgende Vorteile:

— Das hergestellte Glutathion fällt in einer Form an, in der es für die pharmazeutische Applikation unmittelbar geeignet ist.
— Es werden keine chemischen Katalysatoren verwendet, die — sofern sie toxisch oder physiologisch unverträglich sind — von dem erzeugten Glutathion abgetrennt werden müssen.
— Der Hefe in wirbelfähiger Form können auch Stoffe zugesetzt werden, die deren Aktivität nicht verändern, obwohl sie die Permeabilität der Zellwände normalerweise stark erhöhen. Bei submerser Fermentation würden derartige Stoffe die Hefe irreversibel schädigen, da der Zellinhalt in das umgebende wässerige Medium ausfliessen würde.
— Die Präkursoren werden besser umgesetzt als bei der submersen Fermentation.
— Das Verfahren ist besonders wirtschaftlich, weil man die benötigte Hefe unabhängig vom erfindungsgemässen Verfahren ohnehin für andere Zwecke unter optimalen Wachstumsbedingungen in technischem Massstab züchtet und hieraus die benötigten Mengen zwecks Umarbeitung zu wirbelfähigen Partikeln abzweigt.
— Das Glutathion wird in bereits vorhandener Hefe erzeugt. Für die dazu notwendigen Stoffwechselvorgänge können optimale Bedingungen gewählt werden, die unabhängig von den Wachstumsbedingungen bei der Züchtung der Hefe sind.
— Das Verfahren gestattetes, Glutathion in einer Ausbeute herzustellen, die bisher nicht erreichbar war.
— Das Verfahren benötigt nur einen mässig grossen Aufwand an Apparaten und Energie.

Das erfindungsgemässe Verfahren wird durch das folgende Beispiel erläutert, ohne hierauf beschränkt zu sein.

*Beispiel*

Handelsübliche Backhefe (Sacch. cerevisiae) mit 33% Trockensubstanz wurde mit 2% hydrophobiertem Kieselgel vermischt und durch ein Drahtgewebe mit 0,5 mm Maschenweite gedrückt. Das Granulat wurde in einen Wirbelbettreaktor gefüllt und mittels eines Stromes feuchter Luft (Geschwindigkeit 0,8 m/s; Temperatur 25° C) bewegt.

Auf die fluidisierten Partikel wurde innerhalb einer Stunde in mehreren Abschnitten die Präkursor-Lösung in einer Gesamtmenge von 0,2 Liter Lösung pro Kilogramm (feuchter) Hefe aufgesprüht. Die wässerige Lösung enthielt

    10 g/l L-Cystein · HCl
    10 g/l Glycin
    10 g/l L-Glutaminsäure
    30 g/l Ammoniumlaktat

Anschliessend wurde während einer weiteren Stunde im Luftstrom bei 25° C inkubiert. Während dieser Zeit wurde insgesamt etwa 0,2 Liter Wasser pro Kilogramm (feuchter) Hefe auf die fluidisierten Partikel aufgesprüht.

Nach einer Verweilzeit von insgesamt 2 Stunden im Wirbelbettreaktor stieg der Anteil der Trockensubstanz der Hefe von 33% auf 45,5%.

Zur Analyse wurde die Hefe extrahiert. Dazu wurde 1 g Hefe in wenig einprozentiger Phosphorsäure suspendiert und anschliessend auf 10 ml aufgefüllt. Die Suspension wurde im Wasserbad 5 min lang auf 60° C erhitzt, und die Hefezellen wurden abzentrifugiert. In dem klaren Überstand wurde der Glutathion-Gehalt enzymatisch mittels Glyoxalase nach Bernt und Bergmeyer bestimmt (H. U. Bergmeyer: Methoden der enzymatischen Analyse, Verlag Chemie, Weinheim; 3. Auflage (1974), Seiten 1687-1692).

Der Gehalt an Glutathion in der Hefe war von 0,45% auf 2,4% gestiegen, jeweils bezogen auf die Trockensubstanz der Hefe, d.h. auf etwa das Fünffache des normalen Gehalts.

## Patentansprüche

1. Verfahren zur biologischen Herstellung von Glutathion mittels lebender Hefe (Sacch. cerev.) unter Verwendung von L-Cystein, L-Glutaminsäure und Glycin als Präkursoren, gekennzeichnet durch
— Granulieren der Hefe nach bekannten Verfahren und
— Fluidisieren der Hefe mittels Luft im Wirbelbettreaktor und
— Besprühen der fluidisierten Hefe mit einer wässerigen Lösung der Präkursoren und
— weiteres Fluidisieren der Hefe mittels Luft bei einer Temperatur von 20 bis 45° C und einer Verweilzeit von 30 Minuten bis 5 Stunden im Wirbelbettreaktor.

2. Verfahren nach Anspruch 1, gekennzeichnet durch
— Verwenden der behandelten Hefe nach beendeter Umsetzung ohne weitere Aufarbeitung oder
— Konservieren der Hefe in feuchtem Zustand durch Zugaben von bekannten Konservierungsmitteln oder
— Konservieren der Hefe durch Trocknen oder
— Extrahieren des Glutathions aus der Hefe.

## Claims

1. A process for biological production of glutathione by fermenting yeast (Sacch. cerev.) with L-cystein, L-glutamic acid and glycine as precursors, comprising

— granulating the yeast by one of the known procedures, and

— fluidizing the yeast by air within a fluidized bed reactor, and

— spraying the fluidized yeast with an aqueous solution of the precursors, and

— further fluidizing the yeast by air at a temperature from 20 to 45° C over an average particle residence time in the reactor from 30 min to 5 h.

2. A process of Claim 1, comprising

— directly using the treated yeast after completion of the reaction without further processing, or

— adding thereto customary preservatives effective for preserving the yeast in the wet state, or

— drying the yeast for preserving it in the dry state,or

— extracting said glutathione from the yeast.

**Revendications**

1. Procédé pour la production biologique de glutathion au moyen de levure vivante (Sacch. cerev.) par utilisation de L-cystéine, d'acide L-glutamique et de glycine comme précurseurs, caractérisé par:

— la granulation de la levure selon des procédés connus, et

— la fluidification de la levure avec de l'air dans un réacteur à lit fluidisé, et

— l'arrosage de la levure fluidisée avec une solution aqueuse des précurseurs, et

— la poursuite de la fluidification de la levure avec de l'air à une température de 20 à 45° C et pendant une durée de 30 min à 5 h dans le réacteur à lit fluidisé.

2. Procédé selon la revendication 1, caractérisé par:

— l'utilisation de la levure traitée après transformation sans autre traitement, ou

— la conservation de la levure à l'état humide par adjonction de conservateurs connus, ou

— la conservation de la levure par séchage, ou

— l'extraction du glutathion de la levure.